# EUROPEAN PATENT APPLICATION

(11) **EP 2 436 324 A1**
(43) Date of publication of application: **04.04.2012**
(21) Application number: 10183359.8
(22) Date of filing: 30.09.2010
(51) Int. Cl.: A61B 17/66

(54) **Instrument for surgical interventions, particularly orthopedic surgery**

(71) Applicant: Amenduni Gresele, Massimo, 36100 Vicenza (IT)
(72) Inventor: Zona, Mauro, 10153, Torino (IT); Giglio, Gennaro, 80030, Camposano (Napoli) (IT)
(74) Representative: Notaro, Giancarlo

(57) **Abstract**

An instrument (1, 200) for use during a surgical intervention, particularly orthopedic surgery, arranged for application to a limb (L) or another part of the body of a patient that has suffered a bone fracture, comprising:
- a first and a second holding member (4, 6; 204, 206) arranged to be engaged, respectively, on a first and a second portion (L1, L2; B1, B2) of said limb or another part of the body of a patient that has suffered a fracture,
- an actuator (2) comprising a body (10) and a stem (12) moveable with respect to said body (10), wherein said first holding member (4, 204) is connected to said body (10) and said second holding member (6, 206) is connected to said stem (12),
- an electric motor (26) coupled to a transmission (18, 40, 42) operatively connected to said stem (12),
- sensor means (S) for sensing the position of said stem (12),
- electronic control means (CU) operatively connected to said electric motor (26) and cooperating with said sensor means (S),

wherein said electric motor (26) is controllable through said electronic control means (CU) to vary in an accurately controlled manner the relative position of said first and second portion (L1, L2; B1, B2) of said limb or another part of the body of a patient on which said first and second holding member (4, 6; 204, 206) are engaged.

## Description

### Field of the invention

The present invention refers to instruments for surgical interventions, particularly for orthopedic surgery. In particular, the present invention regards an instrument to be used during a surgical intervention for facilitating the repositioning of the bone segments of a limb or another part of the body that has suffered a bone fracture.

### General technical drawback

Following a fracture of a limb or another part of the human body (for example the mandible or the hip bone) the situation is typically that of two or more bone segments separated and misaligned with respect to each other.

In more serious cases, such as for example that of a displaced fracture, the situation is that of a succession of two or more discontinuous bone segments, misaligned with respect to each other and considerably displaced with respect to the normal position thereof, i.e. with respect to the position they would occupy were the bone to which they belong be whole.

This is clear in particular for those bone segments corresponding to portions located in central areas of the bone, i.e. not immediately adjacent to the joints between which the bone is comprised.

In order to guarantee the correct consolidation of the bone segments that had originated following the bone fracture it is necessary, as known, to move the bone segments, by generally applying a traction force along the limb or the part of the body within which the fracture occurred with the aim of creating, so to speak, an axial clearance between the various segments of the fractured bone which is enough for the realignment thereof and reinsertion thereof into the correct position.

As a matter of fact, often the distance between two articular nodes of a limb (or another part of the human body, where such configuration can be found) is reduced following a fracture given that there lacks the support action provided by the bone, hence allowing the ends of the bone segments corresponding to the area of the fracture to overlap slightly.

As known, during a surgical intervention the aid of the hospital personnel, for example one or more nurses assigned to theatre service, are required for applying traction actions to the fractured limb or part of the body with the aim of displacing bone segments generated following the fracture.

The displacement of the bone segments carried out with the help of a manual traction action entails serious problems related to the method through which the entire operation is carried out. As a matter of fact, no matter how much the person performing the traction action is skilled, it is not possible to perform calibrated movements and the patient is also subjected to considerable vibrations and oscillations that are other than negligible and substantially impossible to eliminate.

This means that the result of the consolidation of two or more bone segments strongly depends on factors that are scarcely controllable and reproducible.

The previously described condition, evidently, is not desirable both as regards with the surgeon required to perform the intervention and above all as regards with the patient, in that an erroneous realignment or at least an inaccurate alignment of the bone segments may permanently jeopardise the movement abilities of the patient.

### Object of the invention

The object of present invention is that of overcoming the technical drawbacks described previously. In particular, the object of the present invention is that of providing a more precise, accurate and reproducible realignment of the fractured bone segments during a surgical intervention, particularly orthopedic surgery.

### Summary of the invention

The object of the present invention is achieved by means of an instrument having the characteristics which are subject of the following claims, which form an integral part of the technical disclosure herein provided in relation to the invention.

### Brief description of the drawings

The invention will now be described with reference to the attached drawings, purely provided by way of non-limiting example, wherein:
- figure 1 is a perspective view of an instrument according to a first embodiment of the present invention,
- figure 2 is a perspective view of a component indicated with an arrow IIA in figure 1,
- figure 2A is a sectional view according to the line IIB-IIB of figure 2,
- figure 3 is a perspective view substantially corresponding to figure 2 and illustrating the assembly of further components of the instrument according to the first embodiment of the invention,
- figure 4 is a perspective view according to the arrow IV of figure 1,
- figure 5 is a block diagram illustrating the logical and functional interactions between the components of the instrument according to the present invention,
- figure 6 is a perspective view corresponding to figure 1 illustrating the application of the instrument according to the present invention to the body of a patient,
- figure 6A, 6B, 6C, 6D, 6E, 6F illustrate an operative sequence of the instrument according to the invention,
- figure 7 is a perspective view with some components removed for the clarity of an operative variant of the instrument of figure 1,
- figure 8 is a perspective view corresponding to figure 7 but fully assembled,
- figure 9 is a longitudinal sectional view, substantially along a line analogous to line IIB-IIB, of a further variant of the instrument of figure 1,
- figure 10 is a perspective view of a second embodiment of an instrument according to the present invention,

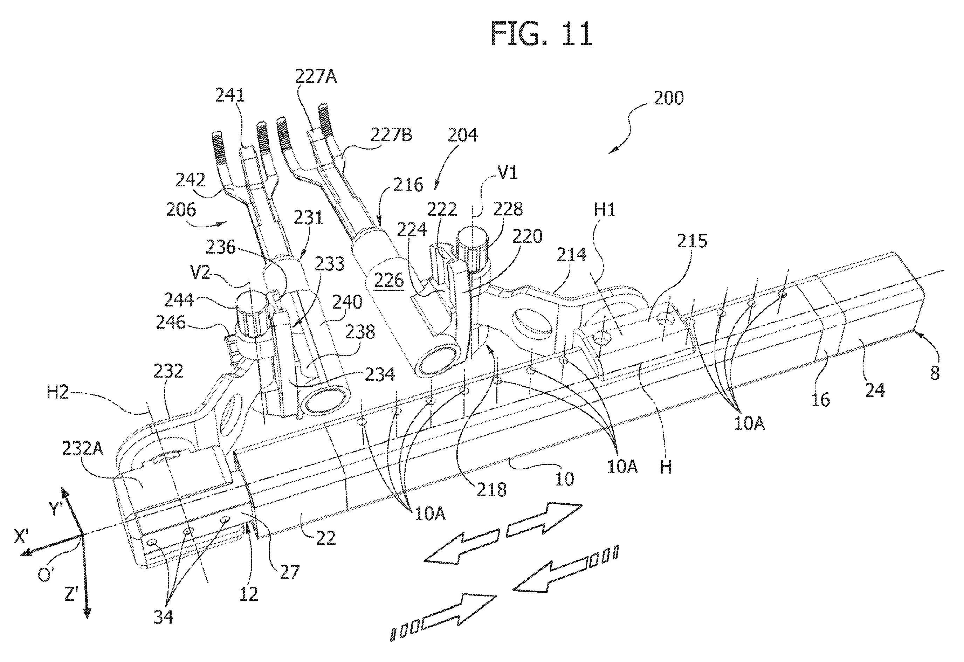

- figure 11 is a perspective view according to the arrow XI of figure 10.

### Detailed description of the invention

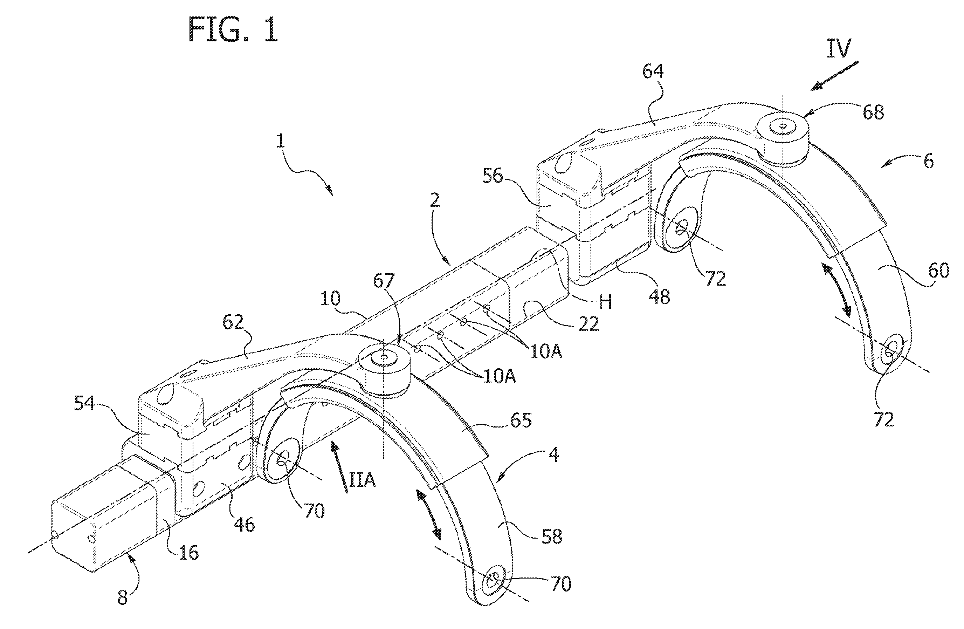

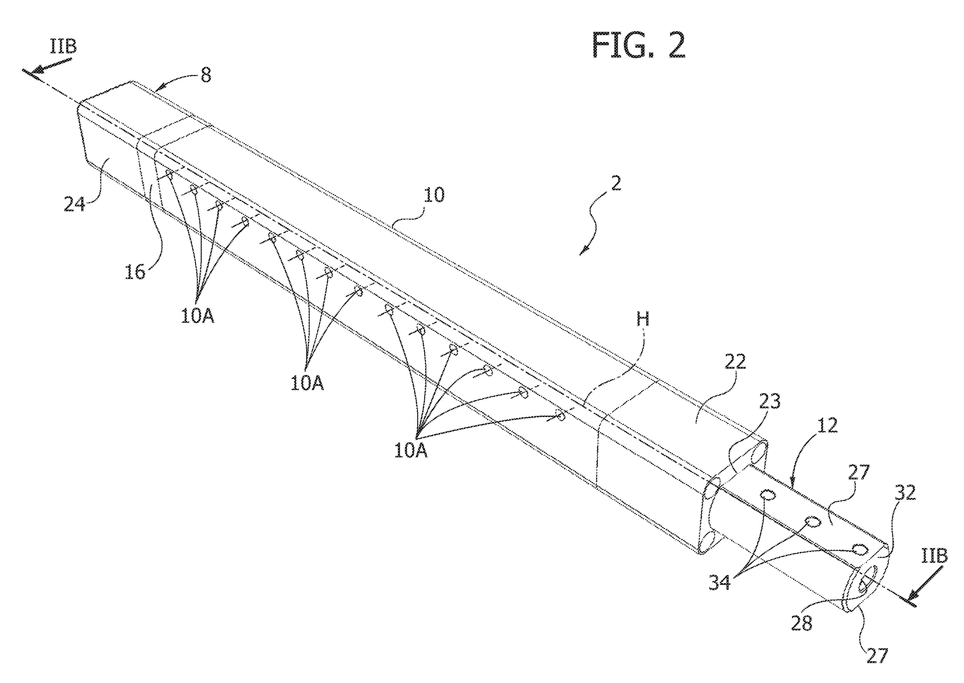

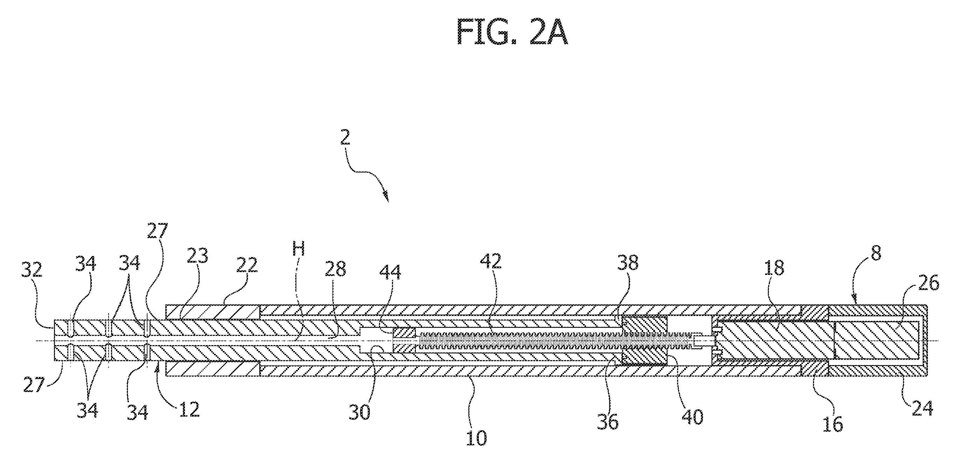

An instrument according to the present invention is indicated with 1 in figure 1. The instrument 1 comprises an actuator 2, a first and a second holding member 4, 6 and a motor unit 8. With reference to figures 2, 2A, the actuator 2 comprises a body 10 and a stem 12 slidable with respect to the body 10.

In this embodiment, the body 10 is provided having a tubular element preferably made of metal material (in particular titanium) having a substantially quadrangular-shaped section and having a longitudinal axis H. A series of threaded holes 10A equally spaced with respect to each other and in which the homologous holes of opposite series are arranged coaxial with respect to each other, are provided on opposite sides of the body 10.

With reference to figure 2A, the body 10 comprises a first end 14 whereat a glass-shaped element 16, preferably made of an aluminium alloy, within which a speed reducer 18 (at times referred to as "reducer 18" for the sake of brevity hereinafter), is fixed. A first guide bushing 22 traversed by a through hole 23 is also fixed at a second end 20 of the body 10. The first guide bushing 22, projects radially towards the internal of the body 10, actually providing an abutment. Such guide bushing is preferably made of polymeric material, for example Iglidur X technopolymer.

The motor unit 8 is fixed to the body 10 at the glass-shaped element 16 and comprises a casing 24 fixed to the glass-shaped container element 16 and an electric motor 26 coupled with the reducer 18. The electric motor 26 has an axis of rotation axis coinciding with the longitudinal axis H.

The stem 12 is partly inside the body 10 and partly projects therefrom traversing the bushing 22; it also has a substantially tubular structure and it is essentially configured as a cylindrical rectilinear element in this embodiment with two longitudinal flattenings 27 parallel and diametrically opposite (hence creating a section whose shape is substantially that of a circumference interrupted by two parallel cords). It should be observed that the section of the through hole 23 of the bushing 22 is substantially identical to the profile of the transverse section of the stem 12 (should the latter be, as described, a circumference interrupted by two parallel cords), so as to prevent the rotation thereof around the axis H (substantially forming a shape coupling).

The stem 12 comprises, in this embodiment, a first through cavity 28 and a second through cavity 30 communicating with respect to each other and both cylindrical shaped. An end of the through cavity 28 faces outside at a first end 32 of the stem 12 (such end is external with respect to the body 10) and it is also in communication with radial threaded holes 34 with axis longitudinal to the axis H and provided along the longitudinal flattenings 27 substantially at the end 32 (six holes 34 are provided in this case, three for each flattening 27).

An abutment 38, to which a nut 40 is fixed (the abutment 38 may possibly be part of a glass-shaped element in which the nut 40 is fixed), is provided for at a second end 36 of the stem 12. A screw 42 is engaged in the nut 40 and it is rotatably connected to an output shaft of the reducer 18. Thus, the electric motor 26 is operatively connected to the stem 12.

The screw 42 is arranged coaxially to the longitudinal axis H and it is arranged substantially inside the through cavity 30. The screw 42 is supported and centred with respect to the cavity 30 by means of a second guide bushing 44 arranged at an end thereof.

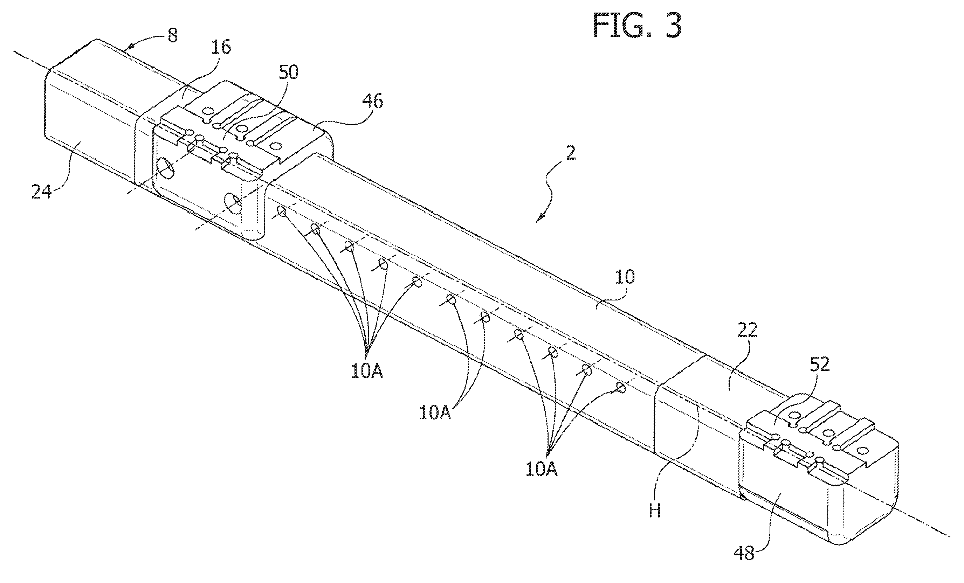

With reference to figure 3, a first and a second fixing element 46, 48 are fixed, respectively, to the body 10 and to the stem 12. In particular, the fixing element 46 fits on the body 10 and it is fixed laterally by means of screws (not illustrated) which are engaged into the threaded holes 10A. Thus, the position of the element 46 may be adjusted with respect to the body 10.

The fixing element 48 instead fits on the stem 12 (astride the flattenings 27) and it is fixed thereto by means of screws (not shown) which are engaged in the radial threaded holes 34. Each of the fixing elements 46, 48 comprises respective cross-shaped formations 50, 52 arranged for coupling with spacer elements, respectively, 54, 56 (preferably made of polymer material) on which the first and the second holding member 4, 6 are fixed respectively. Thus, the first holding member 4 is connected to the body 10, while the second holding member 6 is connected to the stem 12.

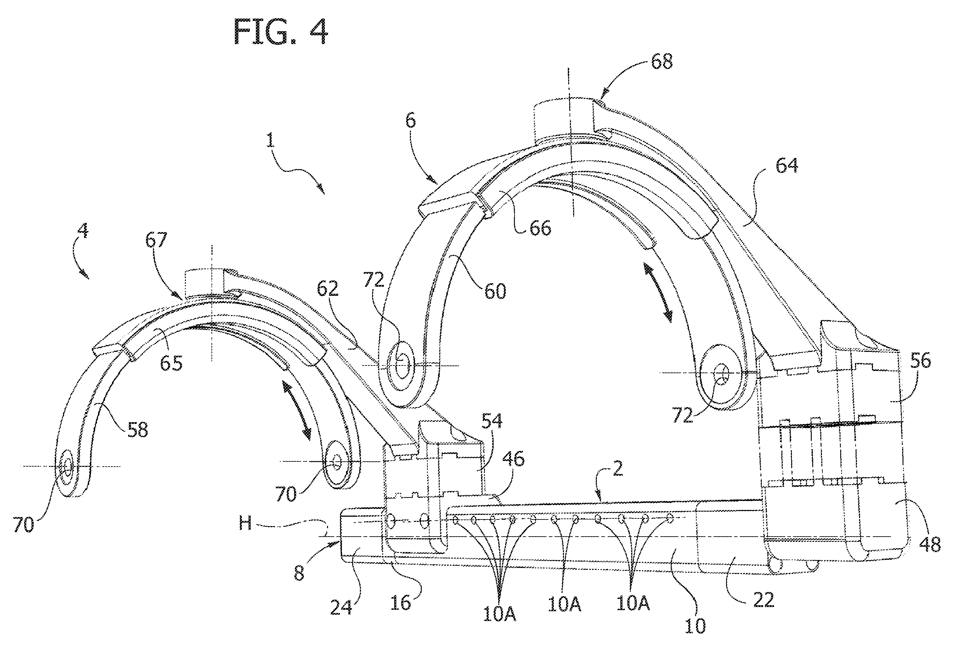

With reference to figures 1 and 4, each holding member 4, 6 comprises a respective substantially C-shaped yoke 58, 60 and a respective bracket 62, 64. Each yoke 58, 60 is received and articulated to the corresponding bracket 62, 64, by means of a respective revolute joint 67, 68, in a respective cradle 65, 66. Thus, each yoke 58, 60 is articulated to the corresponding bracket 62, 64. The yokes 58, 60 are also slidable with respect to the corresponding cradles 65, 66.

Each yoke 58, 60 also comprises a pair of free ends at which coaxial through holes, respectively 70, 72, are provided.

Obviously, the shape of the brackets 62, 64, as well as the type of coupling with respect to each other and the yokes 58, 60 may be widely varied without departing from the scope of the invention. In alternative embodiments, for example, the yokes 58, 60 may be directly rotatably connected, respectively, to the body 10 and to the stem 12 of the actuator 2.

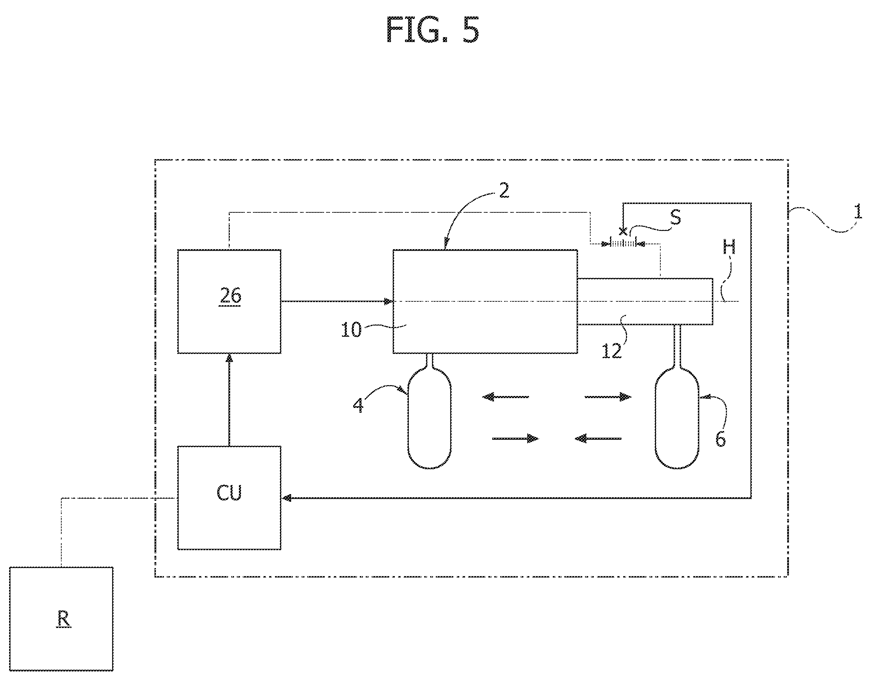

With reference to the schematic representation of figure 5, the instrument 1 comprises an electronic control unit CU operatively connected to the electric motor 26 and cooperating with sensor means for sensing the position of the stem 12 indicated as a whole with S.

A man skilled in the art shall observe that such sensor means may comprise, depending on the specific needs, a linear position transducer of the stem 12 or an angular position transducer (encoder) coupled to the motor 26 or both. Regarding this, it should be observed that the symbol that schematically represents the sensor means S in figure 5 is connected both to the actuator 2 and to the motor 26 indicating the possibility of operative connection of the sensor means S to the actuator 2, to the electric motor 26 or to both.

A remote control unit R is also connected to the instrument 1, and it is operatively connected to the electronic control unit CU for controlling the actuation of the electric motor 26.

The connection arrangement between the electronic control unit CU, the electric motor 26 and the sensor means S is such that a closed loop control of the position of the stem 12 is provided.

The instrument 1 operates as follows.

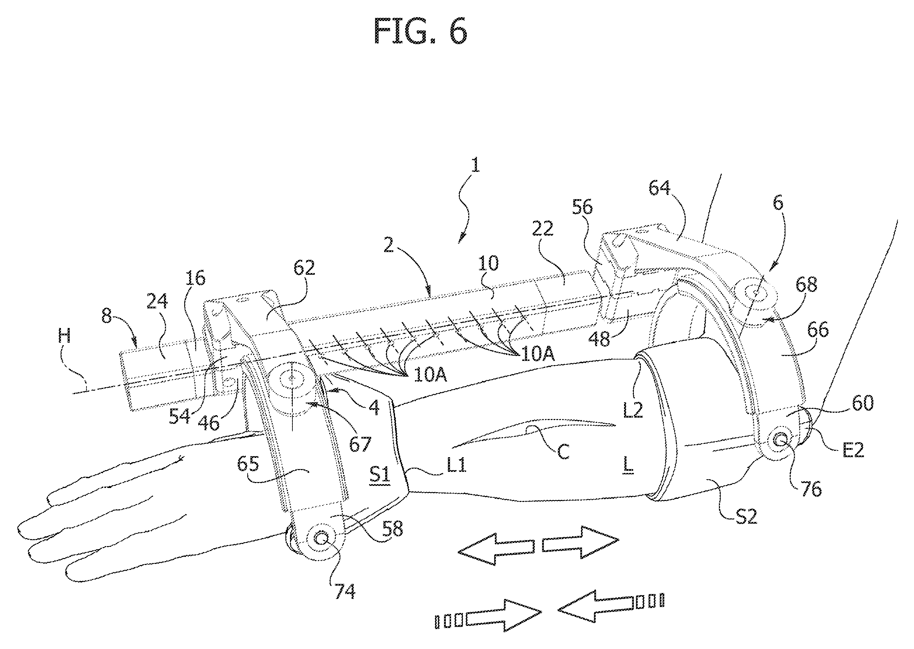

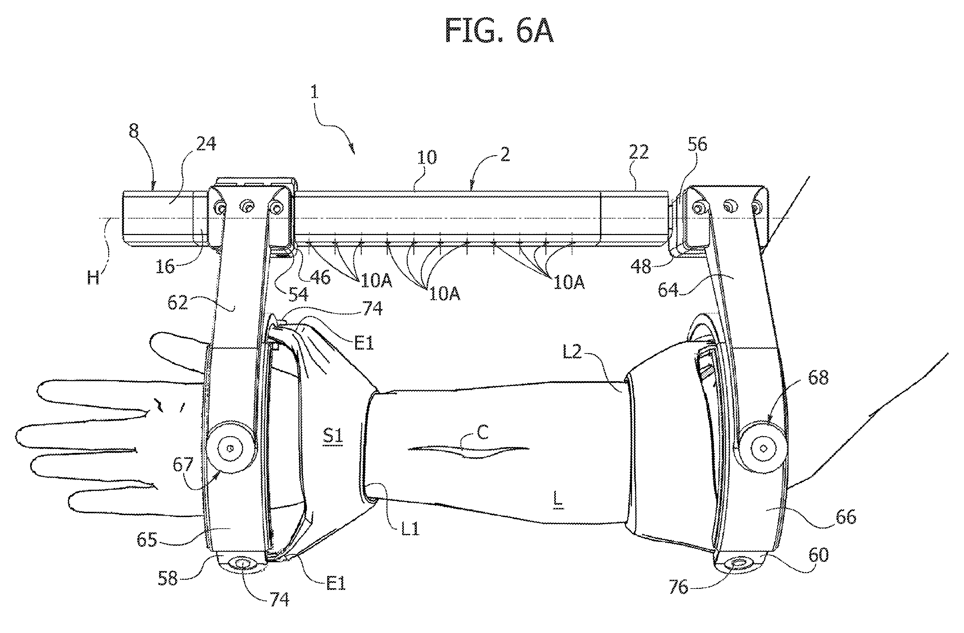

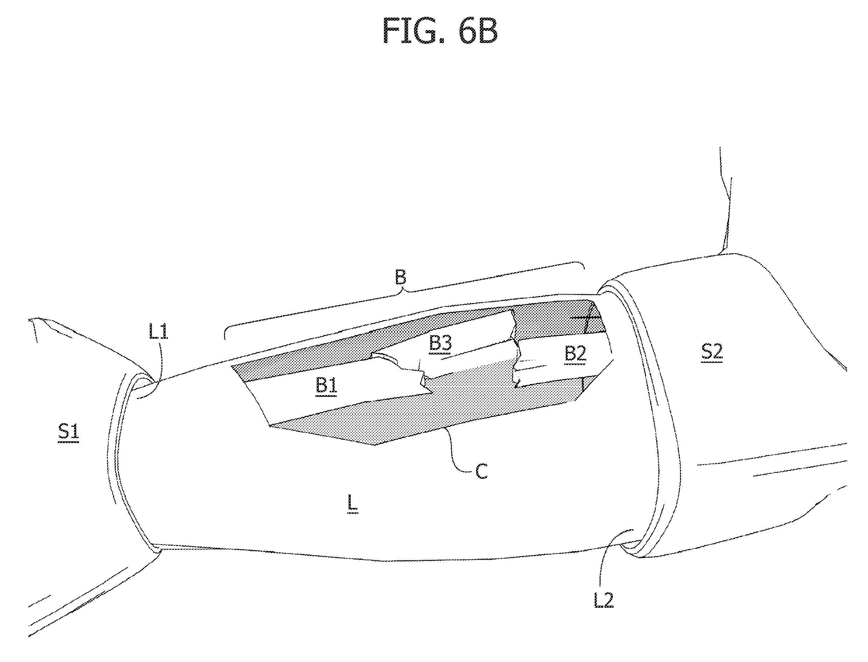

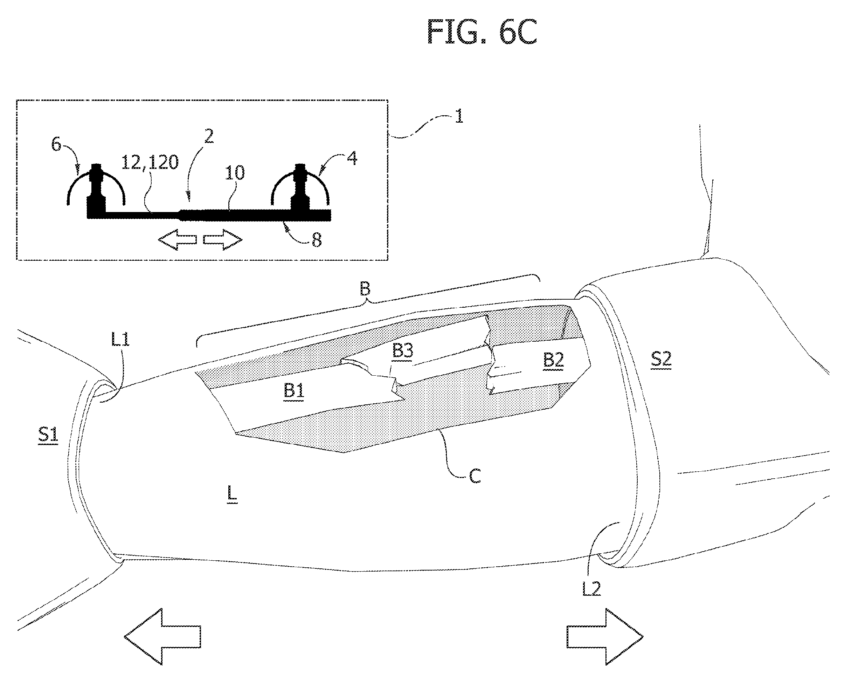

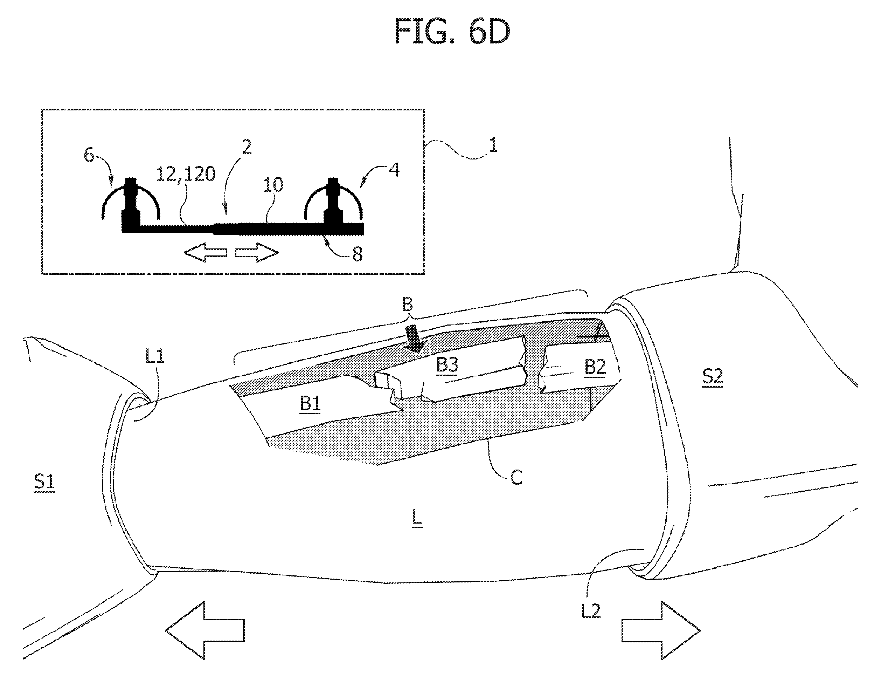

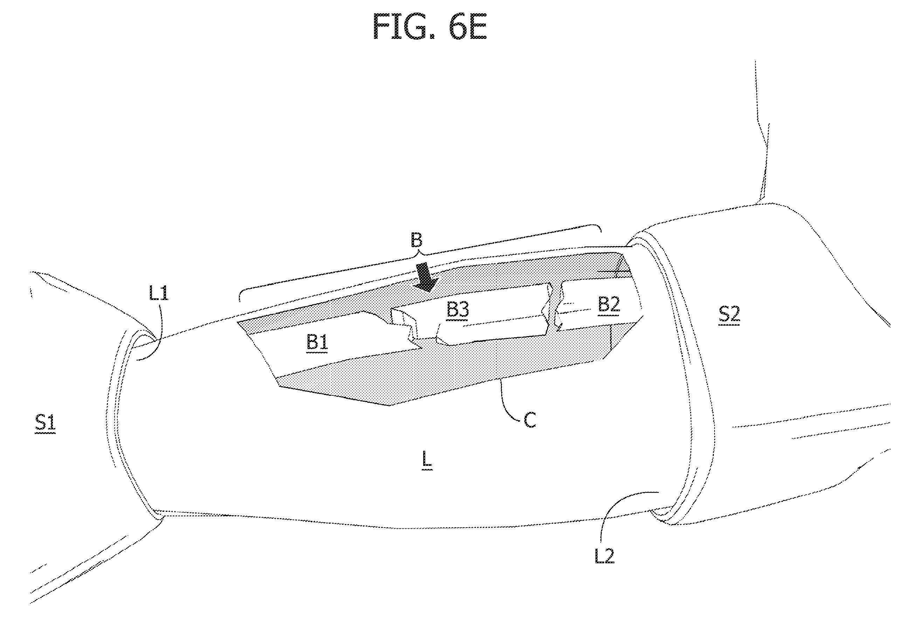

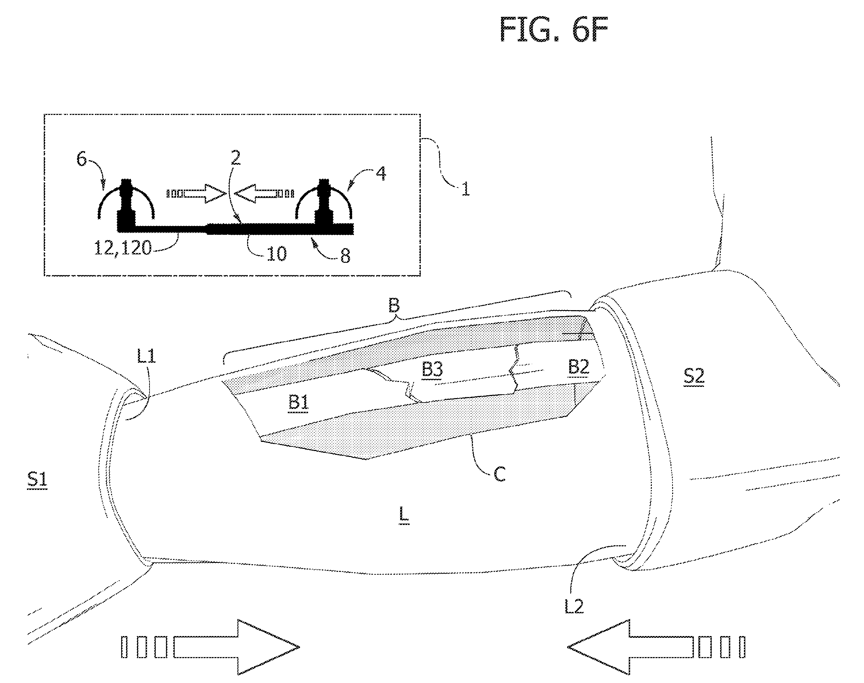

With reference to figure 6, during an orthtopedic surgery intervention the instrument 1 is applied to a limb or generally to a part of the body of a patient that has suffered a fracture.

In figure 6 a fractured limb of a patient (in particular a forearm) is indicated with L, and a first and a second portion of the limb L between which a fractured bone is comprised are indicated with L1 and L2. Each of the portions L1, L2 is wrapped using a respective anatomic strap S1, S2. Thus, in the embodiment described herein, the instrument 1 is substantially an instrument for "external" application, this indicating that the instrument 1 (in particular the holding members 4, 6) is applied externally with respect to the body of a patient (externally with respect to the limb L in this case). This in contraposition with a second embodiment of the instrument 1, which will be described in the following.

Each anatomic strap S1 has the shape of a fabric ring comprising a pair of ears E1, E2 which are received and fixed onto the free ends, respectively, of the yokes 58, 60 fitting on corresponding pairs of pins 74, 76 which traverse the holes 70, 72.

It should be observed that, exploiting the possibility of sliding between the yokes 58, 60 and the corresponding cradles 65, 66 it is possible, as illustrated in figure 6, to adjust the position of the yokes with respect to the limb L (or generally to another part of the body) through the displacement along curvilinear trajectories which reproduce the shapes of the cradles 65, 66 and thus facilitating the positioning of the instrument 1, in particular the actuator 2, with respect to the body of the patient.

The first limb portion L1 is thus connected to the first holding member 4 and to the body 10 of the actuator 2, while the second limb portion L2 is connected to the second holding member 6 and thus to the stem 12 of the actuator 2.

During surgery, the instrument 1 allows varying in an accurately controlled manner (and continuously) the relative position of a holding member with respect to the other. In particular, given that the holding members 4, 6 are connected to parts of the instrument 1 relatively movable with respect to each other, it is clear that the variation of the relative position between the two holding members 4, 6 generates a force on the limb L.

An operative sequence of the instrument 1 is schematically illustrated in figures 6A-F. In some of the figures mentioned above there is a schematic representation of the instrument 1 (enclosed in a square with dash and dot outline) with arrows indicating the direction of displacement of the holding members 4, 6.

Figure 6A illustrates a condition of the beginning of an action for aligning the segments of a fractured bone which substantially corresponds to the condition illustrated in figure 6, i.e. in which the instrument 1 has been applied to the limb L. In addition, it should be observed that, as illustrated in figures 6, 6A, an incision C which allows direct access to the bone segments for possible manipulation thereof is performed on the limb L using other surgical instruments.

With reference to figure 6B, when applying the instrument 1 to the limb L, a bone with a displaced fracture and indicated in its entirety with letter B comprises a first, a second and a third bone segment B1, B2, B3 non continuous and misaligned with respect to each other due to the fracture. In particular, according to what is illustrated in figure 6 the segment B3 is substantially free and considerably misaligned with respect to the segments B1 and B2 which, furthermore, are too close for the segment B3 to spontaneously reinsert therebetween. Furthermore, it should be observed that, were the bone B to be whole, the segments B1, B3, B2 would be continuous with respect to each other in the previously specified sequence. It should also be observed that the representation of the bone B herein provided is purely schematic for the sake of description clarity, without the aim of providing a true reproduction of the anatomy of the human body in the considered area.

With reference to figure 6C, an orthopedic surgeon in charge of performing the intervention may control the extraction of the stem 12 from the body 10 by means of the remote control unit R. The electric motor 26 is controllable through the control unit CU which receives a signal from the remote control unit R and the displacement of the stem 12 is controlled by means of a closed loop logic (figure 5) through the sensor means S, whether a linear position transducer or an encoder (or both) as well as by means of the electronic control unit CU.

The extraction of the stem 12 is obtained, with reference to figure 2A, by rotating the screw 42 in the nut 40. The electric motor 26 controlled by the surgeon by means of the remote control unit R imparts a rotary motion to the screw 42 due to the reducer 18. The rotation of the screw 42, axially fixed, causes an advancement along the longitudinal axis of the nut 40 and of the stem 12 connected thereto. The stem 12, as previously specified, cannot rotate due to the geometry of the through hole 23. During the motion of the stem 12 the screw 42 is, as mentioned, supported and centred with respect thereto, in particular with respect to the through cavity 30, due to the guide bushing 44.

The extraction of the stem 12 with respect to the body 10 causes the holding member 4 to move away with respect to the holding member 6 and thus generates a traction action along the limb L, substantially directed parallel with respect to the longitudinal axis H of the instrument 1. The result of such action is the variation of the relative position of the portions L1, L2 and of the bone segments B1, B2 (and B3, lastly).

With reference to figure 6D, the surgeon may continue the application of a traction action to the limb L simply by operating on the remote control unit R and controlling an increase of the extraction of the stem 12 with respect to the body 10. It should be observed that the action of the instrument 1 on the limb L is, within each movement controlled through the unit R, of the continuous type and the entity thereof may be varied depending on the specific needs, in that the relative position of the holding members 4, 6 is solely decided through the rotation of the motor 26 and the screw 42. This leads to a considerable improvement with respect to what can be obtained through a manual action or using counterweights.

Furthermore, the entity of displacement can be controlled precisely given that there is a direct link (provided by the transmission ratio) between the rotation of the motor 26 and the linear motion of the stem 12 with respect to the body 10.

When a distance between the segments B1 and B2 enough for the insertion of the segment B3 therebetween is achieved, it is possible (figures 6D and 6E) to correctly position the segment B3 for example with the help of a surgical clamp or any other similar handling instrument. Furthermore, it should be observed that the maximum stroke of the stem 12 with respect to the body 10 is limited by the contact between the abutment 38 and the first guide bushing 22.

Upon achieving the ideal alignment (figure 6E), the retraction of the stem 12 into the body 10 can be controlled by simply intervening on the remote control unit R and controlling, through the electronic control unit CU, a rotation of the motor 26 in the opposite direction with respect to the previous actuations.

In such case, the traction action on the limb L is gradually released and the bone segments B1 and B2 are gradually approached to the segment B3 until they reach the condition illustrated in figure 6F wherein the three segments B1, B3, B2 are correctly aligned and coupled with respect to each other. Then it is possible to close the incision C and perform the usual medications, including the application of an orthopaedic cast on the limb L.

Thus, instrument 1 according to the invention has several advantages.

The electric motor 26 can be controlled - through the electronic control unit CU - to vary in an accurately controlled manner the relative position of the first and the second portion L1, L2 of the limb L (or more generally of a part of the body of a patient that has suffered a fracture) and hence of the bone segments B1, B2. The definition "accurately controlled" is used to indicate the fact that the surgeon performing the surgery is always precisely aware of the movement of the stem 12 and thus accurately controls the position of the portions L1, L2 on which the instrument 1, in particular the holding members 4, 6, is applied.

The positioning accuracy of the holding members and, definitely, of the bone segments, guaranteed by the actuator 2 and by the closed loop control reduces the risk of lesions to the patient due to excessive force or positioning errors typical of the manual pulling and displacement of the bone segments.

The progressiveness in the application of the traction action to the limb L guaranteed by the mechanical transmission between the motor 26 and the screw 42 (comprising the reducer 18, the nut 40 and the screw 42 itself) also ensures that there are no impulsive actions or extremely irregular forces during the operation of the instrument 1, all this leading to the advantage of the patient's capacity to bear the treatment.

Furthermore, the presence of health personnel dedicated to the application of a traction force to a fractured limb for the displacement of the bone segments therewithin is no longer required, thus allowing more room for manoeuvre within the theatre, all this leading to the advantage of safety and success of the intervention.

Advantageously, the entire instrument 1 may be sterilised before use, including the electric motor 26. This does not require the application of any sterile cloth for the separation of the sterilised parts from the unsterilized parts, leading to the advantage of simplifying the surgical intervention. Furthermore, the actuator 2 may serve as a support for other instruments usually used in the theatre during orthopedic surgery. For example, the actuator 2 and the instrument 1 may together serve as a support for the various types of surgical instruments, probes, aspirators or ducts.

Furthermore, the instrument 1 can be adapted to a wide variety of limbs or parts of the body that have suffered a fracture, essentially given that the initial position of the holding members 4, 6 may be selected depending on the needs by simply varying the position of the element 46 on the body 10 due to the holes 10A. Furthermore, the revolute joints 67, 68 by means of which the yokes 58, 60 are articulated with respect to the brackets 62, 64 allow compensating possible diversions or particular geometries of the limb or of the part of the body on which the intervention is performed. In addition, the shape and the structure of the brackets 62, 64 illustrated herein shall not be deemed restrictive in any manner whatsoever: depending on the part of the body of the patient on which the instrument 1 is to be applied, the brackets 62, 64 can be replaced with two equivalent elements but of different shape, which can thus better adapt to the anatomy of the human body. It is also possible to eliminate the brackets 62, 64 and provide the revolute joints 67, 68 by directly connecting - in an articulated manner - the body 10 and the stem 12 to the yokes, respectively, 58, 60.

However, extension elements of various lengths can be used should the distances between the holding members 4, 6, - with the stem entirely retracted - not be sufficient for application to a patient (i.e. it is not sufficient, for example, for covering the distance between two articular nodes without causing an extension of the stem thus not exploiting a useful part of the stroke thereof).

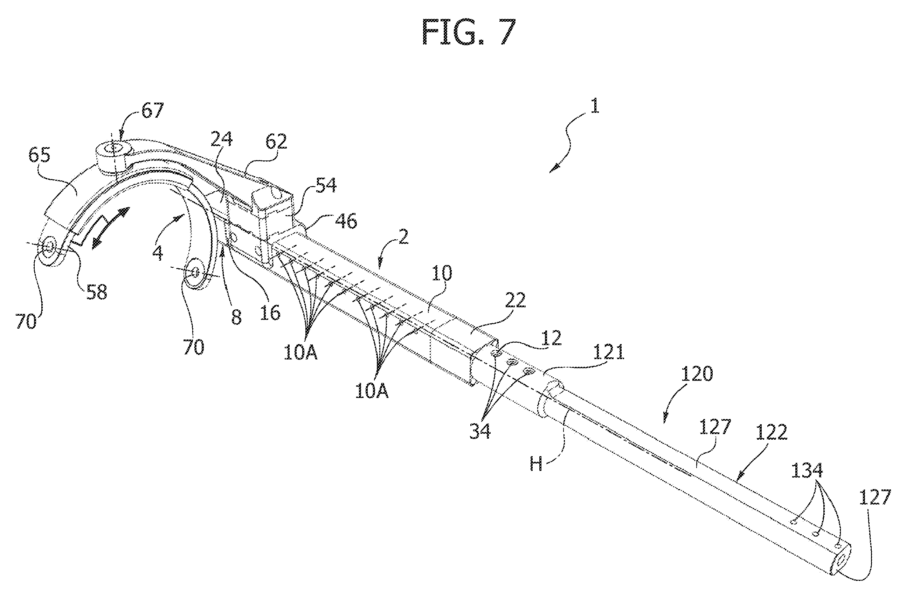

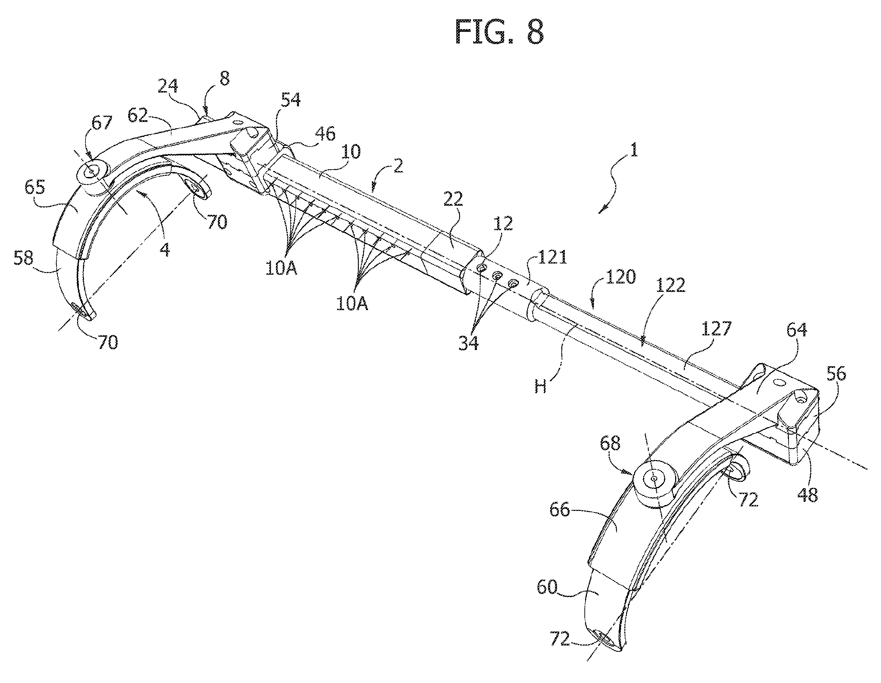

For such purpose, reference shall be made to figures 7, 8, which illustrate the application of an extension to the instrument 1.

All components identical to those illustrated previously are indicated using the same reference number. An extension 120 having an end sleeve 121, fitting onto the stem 12 and fixed thereto by means of screws which are engaged in the threaded holes 34, and an auxiliary stem 122 which reproduces the shapes of the stem 12, comprising that of the end 32 is applied at the end 32 of the stem 12, instead of the connection element 48.

More in detail, the auxiliary stem 122 comprises two longitudinal flattenings 127 at which threaded holes 134 - similar to those of the holes 34 on the stem 12 in terms of number, position and arrangement - are provided. The extension 120 is arranged for fixing the connection element 48 thanks to the holes 134, of the spacer element 56 and of the holding member 6, as illustrated in figure 8. The extension 120 may be provided at various sizes depending on the application, i.e. of the limb or of the part of the body on which the instrument 1 is to be applied, by simply varying the length of the auxiliary stem 122.

Obviously, there is no other variation in the structure and in the operation of the instrument 1, in the mode of interaction with the limb L (or any other limb or part of the body) and in the modalities of displacement of the bone segments.

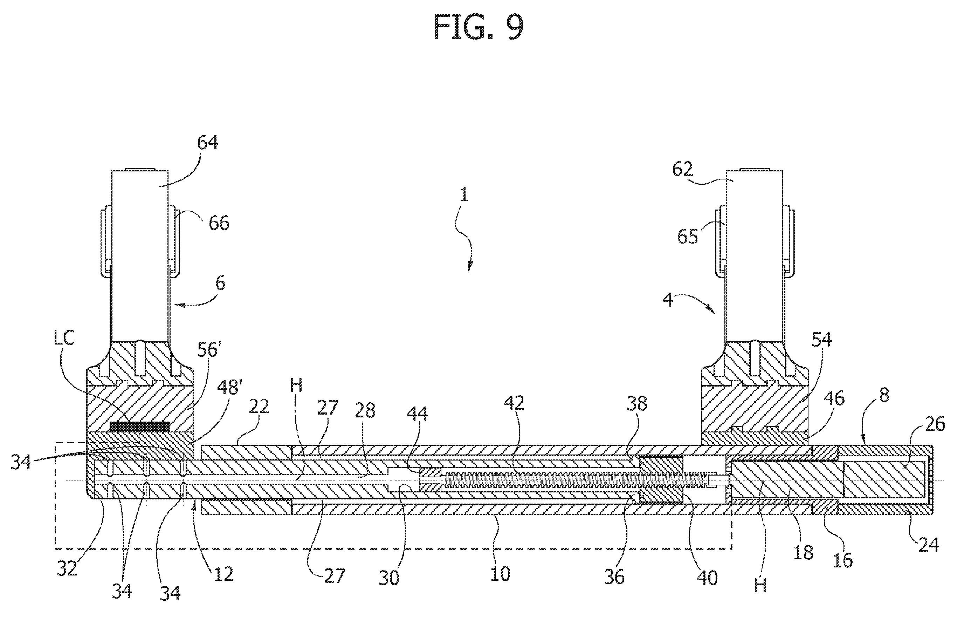

With reference to figure 9, with the aim of guaranteeing better safety to the patient, the instrument 1 may be provided with a load cell LC.

In the embodiment herein illustrated, the load cell LC may be installed on board the instrument 1 by slightly modifying the fixing elements 46, 48 and the spacer elements 54, 56. In the example of figure 9 the fixing element 48 is replaced by a fixing element 48' without the cross-shaped formations 52 to which the load cell LC is fixed. Analogously, the spacer element 56 is replaced by a spacer element 56' also without cross-shaped formations and coupled to the load cell LC. Then, the bracket 64 is normally installed on the element 56'.

Thus, the bracket 64 is coupled to the stem 2 solely by means of the load cell LC, which supports the entire load acting on the holding element 6 (which, for the balance of the system, has the same module with respect to that supported by the holding element 6 and, above all, with respect to the force acting along the limb). Thus, the load cell LC measures the force operating on the limb L by measuring the load operating on the actuator 2 and it is possibly capable of sending a signal which interrupts the operation of the instrument 1 should a traction force deemed harmful to the patient be exceeded. Such threshold value may be adjusted depending on the limb or the part of the body on which the instrument 1 is applied.

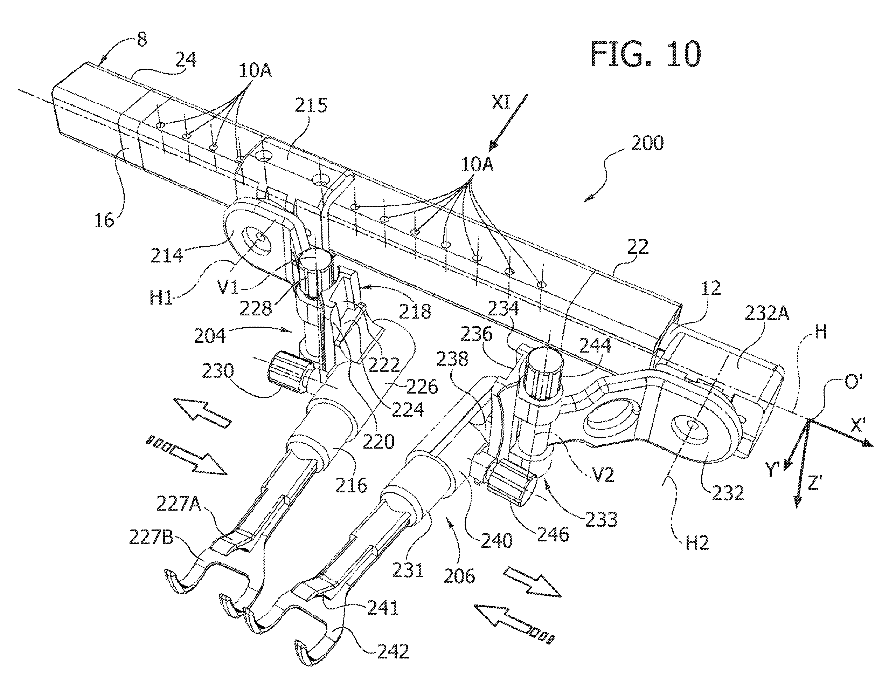

With reference to figures 9, 10, the reference number 200 indicates an instrument to be used during a surgical intervention, particularly orthopedic surgery, according to a second embodiment of the present invention. The components already described previously and identical to those of the instrument 1 are indicated using the same reference number.

The instrument 200 comprises the actuator 2, a first and a second holding member 204, 206 and the motor unit 8.

Furthermore, the diagram of figure 5 applies without distinction also to the instrument 200, simply by replacing the latter to the instrument 1, therefore also the instrument 200 comprises a control unit CU operatively connected to the electric motor 26 and cooperating with sensor means S for sensing the position of the stem 12. The remote control unit R is also provided.

A cartesian reference system O' of the right-hand type including a first axis X' coincident with the longitudinal axis H of the actuator 2, a second axis Y' orthogonal to the axis X' and a third axis Z' orthogonal to the axes X' and Y' and parallel to the axes of the holes 10A is introduced for further clarity of the description.

The holding member 204 comprises a first bracket 214 rotatably connected to the body 10 of the actuator 2, due to a first fixing element 215 (whose position is adjustable by exploiting the coupling with the holes 10A), around an axis H1 parallel to the axis Y' and a first Lowman clamp 216 connected to the bracket 214 by means of a first adjustment unit 218.

The adjustment unit 218 comprises a first swinging guide 220 articulated with respect to the bracket 214 around an axis V1 parallel to the axis Z' and comprising a first circumferential arc-shaped track 222 in which a first slider 224 - rigidly connected to a first tubular element 226 in which the Lowman clamp 216 is inserted - is engaged and slidable. As known to a man skilled in the art, the Lowman clamp 216 comprises a first and a second clamping member 227A, 227B - substantially curve-shaped - arranged for holding a bone or a bone segment.

A first knob 228 engaged on a threaded element (not shown) is arranged for fastening the swinging guide 220 and the bracket 214, thus locking the relative rotation around the axis V1.

A second knob 230 is instead arranged for controlling the sliding of the slider 224 along the track 222. Due to reasons which will appear more evident in the following, the motion transmission from the knob 230 to the slider 224 is preferably of the unilateral and irreversible type, i.e. only a motion transmission from the knob 230 to the slider 224 is allowed and not vice versa.

The second holding member 206 is entirely identical to the first holding member 204 and it comprises a second bracket 232 rotatably connected to the stem 12 of the actuator 2, by means of a second fixing element 232A, around an axis H2 parallel to the axis H1 and to the axis Y' and a second Lowman clamp 231 connected to the bracket 232 by means of a second adjustment unit 233.

The second adjustment unit 233 comprises a second swinging guide 234 articulated with respect to the bracket 232 around an axis V2 parallel to the axis Z' and comprising a second circumferential arc-shaped track 236 in which a second slide 238 - rigidly connected to a second tubular element 240 into which the second Lowman clamp 231 is inserted - is engaged.

The second Lowman clamp 231 is identical to the clamp 216 and it comprises a respective first and second holding element 241, 242 slidable with respect to each other and substantially curve-shaped (analogous to the holding elements 227A, 227B)

A third and fourth knob 244, 246 arranged on the adjustment unit 233 are structurally and functionally analogous, respectively, to the knobs 228, 230.

The instrument 200 operates as follows.

From an operative point of view, the difference with respect to the instrument 1 essentially lies in two aspects:
- the instrument 200 is used during a surgical intervention not for holding the external portions of a limb (or of another part of the body) at the ends of a fractured bone by applying a traction force, but by directly holding the two bone segments misaligned and possibly overlapped with respect to each other due to a fracture and for displacing them directly; thus, the instrument 200 is configured for "internal" application, in that the holding members, particularly the Lowman clamps, are arranged to be engaged on internal portions of a limb or of a part of the body that has suffered a fracture, i.e. on a first and a second bone segment of a fractured bone,
- the action exerted on the fractured limb (or generally on the part of the body of a patient that has suffered a fracture) is slightly different with respect to the instrument 1 given that instead of applying a traction force from outside, the instrument 200 is arranged for moving away the bone segments on which it is directly engaged, which lastly results in an extension of the limb or of the fractured member.

This due to the holding elements 227A, 227B and 241, 242 whose shape is specifically designed for holding segments of human bones.

Therefore, during an orthopedic surgery intervention, partly referring to the numbering previously used for figures 6A-F, it is necessary to perform an incision C whose width is sufficient for the insertion of the Lowman clamps, one of which is engaged on the bone segment B1, the other on the bone segment B2.

Advantageously, the Lowman clamps may be directed in the planes of the reference system O' (thus with respect to the actuator 2) due to the articulation around the axes V1, V2 and the guides 220, 234. In particular, the rotation around the axes V1, V2 allows adjusting the attitude of the Lowman clamps 216, 231 in the plane X'Y', while the translation in the tracks 222, 236 (controlled by means of the knobs 230, 246) allows adjusting the attitude of the Lowman clamps in the plane Y'2'.

When the optimal direction of the Lowman clamps 216, 231 is achieved, it is sufficient to lock the articulation between the guides 220 and 234 and the respective brackets 214, 232 by fastening the knobs 228, 244 (obviously in case of further adjustment it is sufficient to unlock the articulations by loosening the knobs 228, 244).

Regarding the locking of the slides 224, 236 in the respective guides 220, 234, no action is required given that as mentioned, the transmission of the motion from the knobs 246, 230 to the respective sliders is preferably of the unilateral type.

There are no variations in terms of actuation with respect to the instrument 1, given that also the instrument 200 is controlled by means of the remote control unit R and through the electronic control unit CU. By controlling an extraction of the stem 12, the Lowman clamps 216, 231 firmly hold the bone segments B1 and B2 moving them away as the extraction of the stem 12 with respect to the body 10 progresses.

Thus, similarly to what has been described regarding the instrument 1, it is possible to vary - in an accurately controlled manner - the position of the bone segments B1, B2 on which the Lowman clamps 216, 231 are engaged and reposition a possible bone segment B3 (in case of a displaced fracture) between the segments B1 and B2. Furthermore, two fractured bone segments - in case of a non-segmental fracture - can be realigned easily. Furthermore, when the optimal alignment between the bone segments is achieved, a retraction of the stem 12 may be controlled for gradually approaching the segments together.

The previously described operations occur, similarly to the instrument 1, gaining benefit from the accurate positioning, operational regularity and action controllability of the holding members absolutely unachievable through the manual operations of the type usually performed in the operational theatre.

Furthermore, it is possible to install the load cell LC on the instrument 200 for example by intervening on the fixing elements 215, 232A as described regarding the instrument 1, thus increasing the safety of the patient during the surgical intervention.

Lastly, it should be observed that the Lowman clamps are only one possible choice as regards with the instrument 200, which may generally be provided with any tool for holding bone segments. For example, the Lowman clamps 216, 231 may alternatively be replaced (leaving the other components unvaried, and in particular the tubular elements 226, 240 which receive them and which serve as universal support), by:
- a pair of Farabeuf-Lambotte clamps,
- a pair of Lambotte clamps,
- a pair of Baby-Kern clamps, and
- a pair of Verbrugge clamps.

Such clamps are widely known and reference shall be made to specific literature for description thereof.

Thus, a further advantage of the instrument 200 according to the invention emerges: the tools for handling the bone segments may be varied depending on the preferences of the surgeon, to the advantage of the success of the intervention.

Naturally, the construction details and the embodiments may be widely varied with respect to what has been described and illustrated without departing from the scope of protection of the present invention, as defined by the attached claims.

## Claims

1. An instrument (1, 200) for use during a surgical intervention, particularly orthopedic surgery, arranged for the application to a limb (L) or another part of the body of a patient that has suffered a bone fracture, comprising:
- a first and a second holding member (4, 6; 204, 206) arranged to be engaged, respectively, on a first and a second portion (L1, L2; B1, B2) of said limb or another part of the body of a patient that has suffered a bone fracture,
- an actuator (2) comprising a body (10) and a stem (12) moveable with respect to said body (10), wherein said first holding member (4, 204) is connected to said body (10) and said second holding member (6, 206) is connected to said stem (12),
- an electric motor (26) coupled to a transmission (18, 40, 42) operatively connected to said stem (12),
- sensor means (S) for sensing the position of said stem (12),
- electronic control means (CU) operatively connected to said electric motor (26) and cooperating with said sensor means (S), wherein said electric motor (26) is controllable through said electronic control means (CU) to vary in an accurately controlled manner the relative position of said first and second portion (L1, L2; B1, B2) of said limb (L) or another part of the body of a patient on which said first and second holding members (4, 6; 204, 206) are engaged.

2. The instrument (1, 200) according to Claim 1, wherein said electronic control means comprise a control unit (CU), wherein a remote control unit (R) is operatively connected to said control unit (CU), said remote control unit (R) being actuatable for controlling said electric motor (26) and varying the relative position of said first and second portion (L1, L2; B1, B2) of said limb (L) or another part of the body of a patient.

3. The instrument (1, 200) according to Claim 1, wherein said transmission comprises a speed reducer (18) coupled to said electric motor (26), a screw (42) rotatably connected to an output shaft of said speed reducer (18) and a nut (40), fixed to said stem (12), into which said screw (42) is engaged.

4. The instrument (1, 200) according to Claim 3, wherein said stem (12) is a cylindrical-shaped tubular element with two diametrically opposite and parallel longitudinal flattenings (27).

5. The instrument (1, 200) according to Claim 4, wherein said body (10) is a tubular element comprising, at one end (20) thereof a first guide bushing (22) having a through hole (23) having a substantially identical shape with respect to the profile of a transverse section of said stem (12), said stem (12) traversing said guide bushing (22).

6. The instrument (1, 200) according to Claim 3, wherein said stem (12) comprises a first end (32) whereat threaded radial holes (34) are provided and a second end (36) whereat an abutment (38) is provided, said nut (40) being fixed to said abutment (38).

7. The instrument (1, 200) according to Claim 3, wherein said stem (12) comprises a first and a second through cavity (28, 30) communicating with each other and both cylindrical shaped, said second through cavity (30) having a larger diameter with respect to said first through cavity (28) and being arranged for housing said screw (42).

8. The instrument (1, 200) according to Claim 7, wherein said screw (42) is supported and centred with respect to said second through cavity (30) by means of a second guide bushing (44).

9. The instrument (1) according to Claim 1, wherein said first and second holding member comprise, each:
- a bracket (62, 64), and
- a yoke (58, 60) articulated (67, 68) to said bracket (62, 64),
the bracket (62) of said first holding member (4) being fixed to the body (10) of said actuator, the bracket (64) of said second holding member (6) being fixed to the stem (12) of said actuator (2).

10. The instrument (1) according to Claim 9, wherein each yoke (58, 60) is received in a respective cradle (65, 66) articulated to a corresponding of said brackets by means of a revolute joint (67, 68), each yoke (58, 60) being slidable with respect to the corresponding cradle (65, 66).

11. The instrument (1) according to Claim 9 or 10, wherein each yoke (58, 60) comprises, at free ends thereof, a pair of through holes (70, 72) arranged for the insertion of a pin (74, 76), each yoke (58, 60) being arranged for receiving a respective anatomic strap (S1, S2) by means of coupling (E1, E2) with said pins (74, 76), the anatomic straps (S1, S2) being arranged to be engaged on a corresponding of said first and second portion (L1, L2) of said limb (L) or another part of the body of a patient, said first and second portion (L1, L2) further being external portions of said limb (L) or another part of the body of a patient.

12. The instrument (200) according to Claim 1, wherein said first and said second holding member (204, 206) comprise, each:
- a bracket (214, 232)
- a tool (216, 231) connected to said bracket (214, 232) by means of a respective adjustment unit (218, 233) connected to said bracket (214, 232),
and wherein, additionally,
- the bracket (214) of said first holding member (204) is rotatably connected to the body (10) of said actuator (2),
- the bracket (232) of said second holding member (206) is rotatably connected to the stem (12) of said actuator (2)
- the tools (216, 231) of said first and second holding member (204, 206) are arranged to be engaged, respectively, on a first and a second bone segment (B1, B2) of a fractured bone (B) of a limb or another part of the body of a patient.

13. The instrument (200) according to Claim 12, wherein each adjustment unit (218, 233) comprises:
- a swinging guide (220, 234) articulated to a corresponding of said brackets (214, 232) and comprising a circumferential arc-shaped track (222, 236),
- a slider (224, 238) slidable along said circumferential arc-shaped track (222, 236)
- a tubular element (226, 240) fixed to said slider and arranged for housing said tool (216, 231),
- a first and a second knob (228, 230; 244, 246) for controlling the position of said tool (216, 231).

14. The instrument (200) according to Claim 13, wherein, in each adjustment unit (218, 233), said first knob (228, 244) is arranged for disabling or enabling, alternatively, a relative rotation between said bracket (214, 232) and said swinging guide (220, 234), and wherein said second knob (230, 246) is arranged for the displacement of said slider (224, 238) along said circumferential arc-shaped track (222, 236), the transmission of the motion being solely possible from said second knob (230, 246) to said slider (224, 238).

15. The instrument (200) according to any one of Claims 12 to 14, **characterised in that** said tool is selected among:
- a Lowman clamp (216, 231)
- a Farabeuf-Lambotte clamp,
- a Lambotte clamp,
- a Baby-Kern clamp, and
- a Verbrugge clamp.

16. The instrument (1, 200) according to claim 1, comprising a load cell (LC) arranged for measuring a force acting on said actuator (2).
